# EUROPEAN PATENT APPLICATION

(11) **EP 4 480 962 A1**
(43) Date of publication of application: **25.12.2024**
(21) Application number: 22926849.5
(22) Date of filing: 02.12.2022
(51) Int. Cl.: C07K 14/605, A61K 38/26, A61K 47/54, A61K 9/00, A61P 1/00, A61P 3/00

(54) **LONG-ACTING GLP-1/GLP-2 DUAL AGONIST COMPOUND**

(30) Priority: 18.02.2022 CN 202210150559
(71) Applicant: Chengdu Aoda Biotechnology Co., Ltd., Chengdu, Sichuan 610041 (CN)
(72) Inventor: YU, Haining, Chengdu, Sichuan 610041 (CN); ZHOU, Shuliang, Chengdu, Sichuan 610041 (CN); WANG, Peng, Chengdu, Sichuan 610041 (CN)
(74) Representative: PGA S.p.A., Milano, Succursale di Lugano
(86) International application number: PCT/CN2022/136262
(87) International publication number: WO 2023/155545

(57) **Abstract**

The present invention relates to the field of pharmaceutical synthesis, and provides a long-acting GLP-1/GLP-2 dual agonist compound for preparing a pharmaceutical composition for treating diseases and use of the pharmaceutical composition in the preparation of a medicament for treating at least one of the following diseases. The diseases include type II diabetes, impaired glucose tolerance, type I diabetes, obesity, hypertension, metabolic syndrome, dyslipidemia, cognitive disorder, atherosclerosis, myocardial infarction, coronary heart disease, cardiovascular diseases, stroke, inflammatory bowel syndrome, irritable bowel syndrome, dyspepsia or gastric ulcer, hepatic fibrosis diseases, pulmonary fibrosis, etc.

## Description

This application claims the priority of Chinese Patent Application No. 202210150559.7, filed with the China National Intellectual Property Administration on February 18, 2022, and titled with "LONG-ACTING GLP-1/GLP-2 DUAL AGONIST COMPOUND", the disclosure of which is hereby incorporated by reference in its entirety.

### FIELD

The present disclosure relates to a long-acting GLP-1/GLP-2 dual agonist compound and use thereof, wherein the compound is a class of dual incretin mimetic compound that can activate glucagon-like peptide-1 (GLP-1) and glucagon-like peptide-2 (GLP-2) receptors.

### BACKGROUND

Glucagon-like peptide 1 (GLP-1), a peptide with 37 amino acids in length, can stimulate insulin secretion, protect pancreatic β-cells, and inhibit glucagon secretion, gastric emptying, and food intake, leading to weight loss.

Glucagon-like peptide 2 (GLP-2) is produced and secreted by endocrine cells in the small and large intestines. GLP-2 is rapidly inactivated in the circulation by the cleavage at the amino-terminus under the action of dipeptidyl peptidase IV and metabolized by the kidney.

GLP-2 has the effect of promoting the growth and development of intestinal mucosa and proliferation of intestinal epithelial crypt cells, inhibiting the apoptosis of intestinal mucosal epithelial cells and crypt cells, and causing the increase in the height of small intestinal villi and increase in intestinal weight. GLP-2 also has the effect of regulating intestinal brush border enzyme activity, maintaining the integrity of the mucosal epithelium of the small intestinal tract, and promoting the repair of small intestinal injuries. Therefore, there is a broad prospect for the application of GLP-2 in animal nutrition.

Chinese patent application CN201780074629.2 reports that certain GLP-1/GLP-1 compounds exhibit both GLP-1 and GLP-2 activities and have good therapeutic efficacy in gastrointestinal diseases. It is an object of the present disclosure to develop a derivative with higher biological activity to reduce the clinical dose and corresponding side effects.

### SUMMARY

The present disclosure provides a long-acting GLP-1/GLP-2 dual agonist compound and use thereof, wherein the compound is a class of dual incretin mimetic compound that can activate glucagon-like peptide-1 (GLP-1) and glucagon-like peptide-2 (GLP-2) receptors.

In order to achieve the above object, the present disclosure first provides a compound represented by Formula I, a pharmaceutically acceptable salt thereof, a solvate thereof, a chelate thereof, a non-covalent complex thereof, a prodrug thereof, or any mixture thereof.

His-AA1-Glu-Gly-AA2-AA3-AA4-Ser-Glu-Leu-Ala-Thr-AA5- Leu-Asp-AA6(R)-AA7-Ala-Ala-Arg-Asp-Phe-Ile-Ala-Trp-Leu- Ile-AA8-AA9-Lys-Ile-Thr-Asp-AA10 Formula I

wherein AA1 of Formula I is selected from the group consisting of Aib, Gly, Acpr, Acp, Acpe, and Ach;
AA2 of Formula I is Ser, or Thr;
AA3 of Formula I is selected from the group consisting of Phe, αMePhe, and αMePhe(2F);
AA4 of Formula I is Ser, or Thr;
AA5 of Formula I is selected from the group consisting of Ile, Aib, and αMeLeu; and
AA6 of Formula I is selected from the group consisting of Lys, Dap, Dab, Orn, Dah, Dao, Asp[NH(CH₂)ₘNH], Glu[NH(CH₂)ₘNH], Ada[NH(CH₂)ₘNH], Apm[NH(CH₂)ₘNH], and Asu[NH(CH₂)ₘNH];
wherein, m is an integer selected from 2 to 10;
AA7 of Formula I is selected from the group consisting of Gln, Glu, and Leu;
AA8 of Formula I is Ala, or Gin;
AA9 of Formula I is His, or Thr;
AA10 of Formula I is NH₂, or OH;
R of Formula I is HO₂C(CH₂)ₙ₁CO-(AA11)ₙ₂-(PEGₙ₃(CH₂)ₙ₄CO)ₙ₅-;
wherein, R is not HO₂C(CH₂)ₙ₁CO-(AA11)ₙ₂-(PEG₂CH₂CO)₂-;
n1 is an integer selected from 10 to 20,
n2 is an integer selected from 1 to 5,
n3 is an integer selected from 1 to 30,
n4 is an integer selected from 1 to 5,
n5 is an integer selected from 1 to 5,
n6 is an integer selected from 1 to 10; and
AA11 is selected from the group consisting of yGlu, εLys, β-Ala, γ-aminobutyric acid, and 5-Ava.

The present disclosure further provides a pharmaceutical composition comprising the compound according to the present disclosure, as well as use of the pharmaceutical composition comprising the compound of the present disclosure in the manufacture of a medicament for treating a disease.

Preferably, the disease is selected from the group consisting of type II diabetes, impaired glucose tolerance, type I diabetes, obesity, hypertension, metabolic syndrome, dyslipidemia, cognitive disorder, atherosclerosis, myocardial infarction, coronary heart disease, cardiovascular disease, stroke, inflammatory bowel syndrome, irritable bowel syndrome, dyspepsia or gastric ulcer, liver fibrotic disease, pulmonary fibrotic disease and a combination thereof.

More contents of the present disclosure are described in detail below, or some of them may be illustrated in the embodiments of the present disclosure.

Unless otherwise indicated, the amounts of various components and the reaction conditions used herein may be interpreted as "roughly" or "approximately" in any case. Correspondingly, unless otherwise specified, the numerical parameters quoted herein and in the claims are approximate parameters, and different numerical parameters may be obtained due to differences in standard errors under respective experimental conditions.

When there is disagreement or doubt between the chemical structural formula and the chemical name of a compound herein, the chemical structural formula is used to exactly define the compound. The compound described herein may contain one or more chiral centers, and/or double bonds and the like, and may also exist as stereoisomers, including double bond isomers (such as geometric isomers), optically active enantiomers or diastereomers. Accordingly, any chemical structure within the scope of the description herein, whether part of or the whole structure containing similar structures above, includes all possible enantiomers and diastereomers of the compound, including any of the pure stereoisomers (such as pure geometric isomers, pure enantiomers or pure diastereomers) and any mixture of these isomers. These racemic and stereoisomeric mixtures can also be further resolved into their constituent enantiomers or stereoisomers by those skilled in the art using different separation techniques or chiral molecular synthesis methods.

The compounds represented by Formula I include, but are not limited to, optical isomers, racemates and/or mixtures of these compounds. In the above case, pure enantiomer or diastereomer, such as optical isomer, can be obtained by asymmetric synthesis or resolution of racemates. Resolution of racemates can be accomplished by various methods, such as conventional recrystallization with a resolution-promoting agent, or by chromatography. In addition, compounds represented by Formula I also include cis- and/or trans-isomers with double bonds.

The compound of the present disclosure includes, but is not limited to, the compound represented by Formula I and all various pharmaceutically acceptable forms. The various pharmaceutically acceptable forms include various pharmaceutically acceptable salts, solvates, complexes, chelates, non-covalent complexes, prodrugs based on the aforementioned substances and any mixture of the aforementioned forms.

### DETAILED DESCRIPTION

The present disclosure discloses a long-acting GLP-1/GLP-2 dual agonist compound and use thereof. Those skilled in the art can learn from the content of the present disclosure and appropriately improve relevant parameters for implementation. It should be particularly noted that all similar substitutions and modifications are obvious to those skilled in the art, and they are deemed to be included in the present disclosure. The method of the present disclosure has been described through the preferred embodiments, and it is obvious that those skilled can achieve and apply the techniques of the present disclosure by changes or appropriate modifications and combinations of the compound and the preparation method described herein, without departing from the content, spirit and scope of the preset disclosure.

The terms corresponding to the abbreviations used in the present disclosure are shown in the following table:

| Abbreviation | Name | Abbreviation | Name |
|---|---|---|---|
| Fmoc | 9-Fluorenemethoxycarbonyl | OtBu | Tert-butoxy |
| tBu | Tert-butyl | Boc | Tert-butoxycarbonyl |
| Trt | Trityl | Pbf | (2,3-Dihydro-2,2,4,6,7-pentameth ylbenzofuran-5-yl)sulfonyl |
| Ala | Alanine | Leu | Leucine |
| Arg | Arginine | Lys | Lysine |
| Asn | Asparagine | Met | Methionine |
| Asp | Aspartic acid | Phe | Phenylalanine |
| Cys | Cysteine | Pro | Proline |
| Gln | Glutamine | Ser | Serine |
| Glu | Glutamic acid | Thr | Threonine |
| Gly | Glycine | Trp | Tryptophan |
| His | Histidine | Tyr | Tyrosine |
| Ile | Isoleucine | Val | Valine |
| Aib | Aminoisobutyric acid | Dah | 2,7-diaminoheptanoic acid |
| 5-Ava | 5-Aminovaleric acid | Dao | 2,8-diaminooctanoic acid |
| Dap | 2,3-Diaminopropionic acid | Ada | 2-aminoadipic acid |
| Dab | 2,4-Diaminobutyric acid | Apm | 2-aminopimelic acid |
| Orn | Ornithine | Asu | 2-aminooctanedioic acid |

### Example 1 Preparation of compound

The preparation method comprises: preparing a peptide resin by a solid-phase polypeptide synthesis method, then subjecting the peptide resin to acid hydrolysis to obtain a crude product, and finally purifying the crude product to obtain a pure product; wherein the step of preparing a peptide resin by a solid-phase polypeptide synthesis method is performed by sequentially coupling the protected amino acid or fragment corresponding to the polypeptide sequences to a carrier resin by solid-phase coupling synthesis to prepare the peptide resin.

In the above preparation method, the amount of the Fmoc-protected amino acid or the protected amino acid fragment is 1.2-6 times, preferably 2.5-3.5 times of the total moles of the used resin.

In the above preparation method, the degree of substitution of the carrier resin is 0.2-1.0 mmol/g of resin, preferably 0.3-0.5 mmol/g of resin.

As a preferred embodiment of the present disclosure, the solid-phase coupling synthesis method comprises: removing the Fmoc protecting group from the protected amino acid-resin obtained in the previous step, and subjecting the resulting resin to a coupling reaction with the next protected amino acid. The removal of the Fmoc protecting group is performed for 10-60 min, preferably for 15-25 min. The coupling reaction is performed for 60-300 min, preferably for 100-140 min.

The coupling reaction requires the addition of a condensing agent, and the condensing agent is selected from the group consisting of DIC (N,N-diisopropylcarbodiimide), N,N-dicyclohexylcarbodiimide, benzotriazole-1-yl-oxytripyrrolidinophosphonium hexafluorophosphate, 2-(7-aza-1H-benzotriazole-1-yl)-1,1,3,3-tetramethyluronium hexafluorophosphate, benzotriazole-N,N,N',N'-tetramethyluronium hexafluorophosphate and O-benzotriazole-N,N,N',N'-tetramethyluronium tetrafluoroborate; preferably N,N-diisopropylcarbodiimide. The molar amount of the condensing agent is 1.2-6 times, preferably 2.5-3.5 times, of the total moles of amino groups in the amino resin.

The coupling reaction requires the addition of an activating agent, and the activating agent is selected from the group consisting of 1-hydroxybenzotriazole and N-hydroxy-7-azabenzotriazole, preferably 1-hydroxybenzotriazole. The amount of the activating agent is 1.2-6 times, preferably 2.5-3.5 times, of the total moles of amino groups in the amino resin.

As a preferred embodiment of the present disclosure, a reagent for removing Fmoc protection is a mixed solution of PIP/DMF (piperidine/N,N-dimethylformamide), and the content of piperidine in the mixed solution is 10-30% (V). The amount of the reagent for removing Fmoc protection is 5-15 mL per gram of amino resin, preferably 8-12 mL per gram of amino resin.

Preferably, the peptide resin is subjected to acid hydrolysis to remove both the resin and the protecting group of side chain to obtain a crude product.

Further preferably, an acid hydrolyzing agent used during acid hydrolysis of the peptide resin is a mixed solvent of trifluoroacetic acid (TFA), 1,2-ethanedithiol (EDT) and water, and the volume percentage of each component in the mixed solvent is as follows: TFA 80-95%, EDT 1-10%, and the balance is water.

More preferably, the volume percentage of each component in the mixed solvent is as follows: TFA 89-91%, EDT 4-6%, and the balance is water. The optimal volume percentage of each component in the mixed solvent is as follows: TFA 90%, EDT 5%, and the balance is water.

The amount of the acid hydrolyzing agent used is 4-15 mL of acid hydrolyzing agent per gram of peptide resin; preferably, 7-10 mL of acid hydrolyzing agent per gram of peptide resin.

The acid hydrolysis using the acid hydrolyzing agent is performed for 1-6 hours at room temperature, preferably for 3 to 4 hours.

Further, the crude product is purified by high performance liquid chromatography and freeze-dried to obtain a pure product.

### 1. Synthesis of peptide resin

Rink Amide BHHA resin, as the carrier resin, was subjected to the removal of Fmoc protection, and sequentially coupled with the protected amino acids corresponding to the polypeptide sequences to obtain the peptide resin.

### (1) Coupling a first protected amino acid in main chain

0.03 mol of the first protected amino acid and 0.03 mol of HOBt were dissolved in an appropriate amount of DMF. 0.03 mol of DIC was then slowly added to the DMF solution of the protected amino acid under stirring, stirred and reacted at room temperature for 30 min to obtain an activated solution of the protected amino acid for later use.

0.01 mol of Rink amide MBHA resin (degree of substitution was about 0.4 mmol/g) was subjected to deprotection with 20% PIP/DMF solution for 25 min, washed and filtered to obtain a Fmoc-removed resin.

The activated solution of the first protected amino acid was added to the Fmoc-removed resin for coupling reaction for 60-300 min, filtered and washed to obtain a resin containing the first protected amino acid.

### (2) Coupling the protected amino acids in main chain

The above-mentioned protected amino acids corresponding to the polypeptide sequence were sequentially coupled by the same method as above for coupling the first protected amino acid in the main chain to obtain a resin containing amino acids in the main chain.

### (3) Coupling a first protected amino acid in side chain

0.03 mol of a first protected amino acid in side chain and 0.03 mol of HOBt were dissolved in an appropriate amount of DMF. 0.03 mol of DIC was slowly added to the DMF solution of the protected amino acid under stirring, stirred and reacted at room temperature for 30 min to obtain the activated solution of the protected amino acid.

2.5 mmol of tetrakistriphenylphosphine palladium and 25 mmol of phenylsilane were dissolved in an appropriate amount of dichloromethane, subjected to deprotection for 4 hours, filtered and washed to obtain Alloc-removed resin for later use.

The activated solution of the first protected amino acid in side chain was added to the Alloc-removed resin for coupling reaction for 60-300 min, filtered and washed to obtain a resin containing the first protected amino acid in side chain.

### (4) Coupling other protected amino acids or a monoprotected fatty acid in side chain

The protected amino acids and monoprotected fatty acid corresponding to the side chain were sequentially coupled by the same method as above for coupling the first protected amino acid in main chain to obtain a peptide resin.

### 2. Preparation of crude product

To the above peptide resin, a lysing agent with a volume ratio of TFA: water: EDT=95:5:5 (10 mL of lysing agent/g of resin) was added, stirred evenly and reacted under stirring at room temperature for 3 h. The reaction mixture was filtered with a sand core funnel, the filtrate was collected, and the resin was washed three times with a small amount of TFA. The filtrates were combined, concentrated under reduced pressure, and precipitated with anhydrous ether. The precipitate was washed with anhydrous ether three times, and dried by suction to obtain a crude product as an off-white powder.

### 3. Preparation of pure product

The above crude product was added with water and stirred. The pH of the solution was adjusted to 8.0 with aqueous ammonia until the crude product was completely dissolved. The solution was filtered with a 0.45 µm mixed microporous filtration membrane and purified for later use.

The purification was performed by high-performance liquid chromatography. The chromatographic packing material for purification was 10 µm reverse phase C18. The mobile phase system was 0.1%TFA/aqueous solution-0.1%TFA/acetonitrile solution. The flow rate of the 30 mm*250 mm chromatographic column was 20 mL/min. The elution was performed by a gradient system, and the loading was cycled for purification. The solution of the crude product was loaded into the chromatographic column and then eluted with the mobile phase. The eluate of the main peak was collected and subjected to evaporation to remove acetonitrile to obtain a purified intermediate concentrate.

The purified intermediate concentrate was filtered with a 0.45 µm filter membrane for later use. Salt exchange was performed by high-performance liquid chromatography. The mobile phase system was 1% acetic acid/aqueous solution-acetonitrile. The chromatographic packing material for purification was 10 µm reverse phase C18. The flow rate of the 30 mm*250 mm chromatographic column was 20 mL/min (the corresponding flow rate could be adjusted according to the chromatographic column of different specifications). The gradient elution and cyclic sample loading was performed. The sample was loaded into the chromatographic column and then eluted with the mobile phase. The eluates were collected and subjected to spectrum analysis, and change in absorbance was observed. The eluate of the main peak during salt exchange was collected and detected for purity using analytical liquid chromatography. The eluate of the main peak during salt exchange was combined, and concentrated under reduced pressure to obtain an aqueous acetic acid solution of pure product, which was then freeze-dried to obtain the pure peptide.

The following compounds were synthesized using the methods described above:

| | |
|---|---|
| Compound 1 | His-Aib-Glu-Gly-Ser-Phe-Thr-Ser-Glu-Leu-Ala-Thr-Ile-Leu-Asp- Lys(γGlu-octadecanedioic acid-Gln-Ala-Ala-Arg-Asp-Phe-Ile-Ala-Trp-Leu- Ile-Gln-His-Lys-Ile-Thr-Asp-NH2 |
| Compound 2 | His-Aib-Glu-Gly-Ser-Phe-Thr-Ser-Glu-Leu-Ala-Thr-Ile-Leu-Asp- Lys(PEG1CH2CO-γGlu-octadecanedioic acid)-Gln-Ala-Ala-Arg-Asp-Phe- Ile-Ala-Trp-Leu-Ile-Gln-His-Lys-Ile-Thr-Asp-NH2 |
| Compound 3 | His-Aib-Glu-Gly-Ser-Phe-Thr-Ser-Glu-Leu-Ala-Thr-Ile-Leu-Asp- Lys(AEEA-γGlu-octadecanedioic acid)-Gln-Ala-Ala-Arg-Asp-Phe- Ile-Ala-Trp-Leu-Ile-Gln-His-Lys-Ile-Thr-Asp-NH2 |
| Compound 4 | His-Aib-Glu-Gly-Ser-Phe-Thr-Ser-Glu-Leu-Ala-Thr-Ile-Leu-Asp- Lys(PEG3CH2CO-γGlu-octadecanedioic acid)-Gln-Ala-Ala-Arg-Asp-Phe- Ile-Ala-Trp-Leu-Ile-Gln-His-Lys-Ile-Thr-Asp-NH2 |
| Compound 5 | His-Aib-Glu-Gly-Ser-Phe-Thr-Ser-Glu-Leu-Ala-Thr-Ile-Leu-Asp- Lys(PEG4CH2CO-γGlu-octadecanedioic acid)-Gln-Ala-Ala-Arg-Asp-Phe- Ile-Ala-Trp-Leu-Ile-Gln-His-Lys-Ile-Thr-Asp-NH2 |
| Compound 6 | His-Aib-Glu-Gly-Ser-Phe-Thr-Ser-Glu-Leu-Ala-Thr-Ile-Leu-Asp- Lys(PEG5CH2CO-γGlu-octadecanedioic acid)-Gln-Ala-Ala-Arg-Asp-Phe- Ile-Ala-Trp-Leu-Ile-Gln-His-Lys-Ile-Thr-Asp-NH2 |
| Compound 7 | His-Aib-Glu-Gly-Ser-αMePhe-Thr-Ser-Glu-Leu-Ala-Thr-Ile-Leu- Asp-Lys(PEG3CH2CO-γGlu-octadecanedioic acid)-Gln-Ala-Ala-Arg-Asp- Phe-Ile-Ala-Trp-Leu-Ile-Gln-His-Lys-Ile-Thr-Asp-NH2 |
| Compound 8 | His-Aib-Glu-Gly-Ser-αMePhe(2F)-Thr-Ser-Glu-Leu-Ala-Thr-Ile-Leu- Asp-Lys(PEG3CH2CO-γGlu-octadecanedioic acid)-Gln-Ala-Ala-Arg-Asp- Phe-Ile-Ala-Trp-Leu-Ile-Gln-His-Lys-Ile-Thr-Asp-NH2 |
| Compound 9 | His-Aib-Glu-Gly-Ser-αMePhe-Thr-Ser-Glu-Leu-Ala-Thr-Aib-Leu- Asp-Lys(PEG3CH2CO-γGlu-octadecanedioic acid)-Gln-Ala-Ala-Arg-Asp- Phe-Ile-Ala-Trp-Leu-Ile-Gln-His-Lys-Ile-Thr-Asp-NH2 |
| Compound 10 | His-Aib-Glu-Gly-Ser-αMePhe(2F)-Thr-Ser-Glu-Leu-Ala-Thr-Aib-Leu- Asp-Lys(PEG3CH2CO-γGlu-octadecanedioic acid)-Gln-Ala-Ala-Arg-Asp- |
| | Phe-Ile-Ala-Trp-Leu-Ile-Gln-His-Lys-Ile-Thr-Asp-NH2 |
| Compound 11 | His-Aib-Glu-Gly-Ser-αMePhe-Thr-Ser-Glu-Leu-Ala-Thr-αMeLeu-Leu- Asp-Lys(PEG3CH2CO-γGlu-octadecanedioic acid)-Gln-Ala-Ala-Arg-Asp- Phe-Ile-Ala-Trp-Leu-Ile-Gln-His-Lys-Ile-Thr-Asp-NH2 |
| Compound 12 | His-Aib-Glu-Gly-Ser-αMePhe(2F)-Thr-Ser-Glu-Leu-Ala-Thr-αMeLeu- Leu-Asp-Lys(PEG3CH2CO-γGlu-octadecanedioic acid)-Gln-Ala-Ala-Arg-Asp- Phe-Ile-Ala-Trp-Leu-Ile-Gln-His-Lys-Ile-Thr-Asp-NH2 |

### Example 2 Determination of GLP-1 activity

### 1. Determination method

GLP-1R, under the stimulation of its specific agonist, can activate the intracellular adenylate cyclase pathway, and increase the level of cAMP, finally leading to the generation and release of insulin. The stimulation of the cell lines stably transfected with GLP-1R by the agonist to be tested can lead to rapid increase in the intracellular cAMP level, and the relative light units (RLUs) after stimulating the cells at each dose are determined by chemiluminescence method to calculate the EC50 of the agonist. This method for determining activity is commonly used for GLP-1 receptor agonist activity analysis at home and abroad.

CHO-K1 cell line stably expressing GLP-1R was stimulated with the agonist at different concentrations. The EC₅₀ value of the agonist was calculated by determining the relative light units after stimulating the cells at each dose.

### 2. Determination results

The determination results are shown in the table below.

| **Compound** | **GLP-1 activity [EC₅₀ (pmol)]** |
|---|---|
| Compound 1 | 94.66 |
| Compound 2 | 68.65 |
| Compound 3 | 60.14 |
| Compound 4 | 60.00 |
| Compound 5 | 72.80 |
| Compound 6 | 79.45 |

### Example 3 Determination of GLP-2 activity

### 1. Determination method

GLP-2R, under the stimulation of its specific agonist, can activate the intracellular adenylate cyclase pathway, and increase the level of cAMP, finally leading to the generation and release of insulin. The stimulation of the cell lines stably transfected with GLP-1R by the agonist to be tested can lead to rapid increase in the intracellular cAMP level, and the relative light units (RLUs) after stimulating the cells at each dose are determined by chemiluminescence method to calculate the EC50 of the agonist. This method for determining activity is commonly used for GLP-2 receptor agonist activity analysis at home and abroad.

CHO-K1 cell line stably expressing GLP-2R was stimulated with the agonist at different concentrations. The EC₅₀ value of the agonist was calculated by determining the relative light units after stimulating the cells at each dose.

### 2. Determination results

The determination results are shown in the table below.

| **Compound** | **GLP-2 activity [EC₅₀ (nmol)]** |
|---|---|
| Compound 1 | 6.04 |
| Compound 2 | 5.51 |
| Compound 3 | 4.70 |
| Compound 4 | 4.33 |
| Compound 5 | 5.54 |
| Compound 6 | 4.62 |

### Example 3 Preliminary determination of pharmacokinetic properties

The test animals were cynomolgus monkeys, two male cynomolgus monkeys in each compound group, and the drug was subcutaneously administered at 0.1 mg/kg. The blood was collected intravenously before (0 h) the administration, and 1h, 2h, 3h, 4h, 8h, 12h, 18h, 24h, 48h, 96h, 144h, and 168h after the administration of the compounds, and then centrifuged to isolate plasma samples. Then the serum drug concentration of corresponding compound in the plasma sample was determined by liquid chromatography-mass spectrometry, and the half-life of the compound after subcutaneous (SC) administration is shown in the table below.

| **Compound** | **t_{1/2} (h)** |
|---|---|
| Compound 4 | 59.2 |

## Claims

1. A compound represented by Formula I:
His-AAI-Glu-Gly-AA2-AA3 -AA4-Ser-Glu- Leu- Ala- Thr-AA5 -Leu- Asp-AA6(R)-AA 7 - Ala- Ala-Arg-Asp-Phe-Ile-Ala-Trp-Leu-Ile-AA8-AA9-Lys-Ile-Thr-Asp-AA10 Formula I
wherein AA1 of Formula I is selected from the group consisting of Aib, Gly, Acpr, Acp, Acpe, and Ach;
AA2 of Formula I is Ser, or Thr;
AA3 of Formula I is selected from the group consisting of Phe, αMePhe, and αMePhe(2F);
AA4 of Formula I is Ser, or Thr;
AA5 of Formula I is selected from the group consisting of Ile, Aib, and αMeLeu; and
AA6 of Formula I is selected from the group consisting of Lys, Dap, Dab, Orn, Dah, Dao, Asp[NH(CH2)mNH], Glu[NH(CH2)mNH], Ada[NH(CH2)mNH], Apm[NH(CH2)mNH], and Asu[NH(CH₂)ₘNH];
wherein, m is an integer selected from 2 to 10;
AA7 of Formula I is selected from the group consisting of Gln, Glu, and Leu;
AA8 of Formula I is Ala, or Gln;
AA9 of Formula I is His, or Thr;
AA10 of Formula I is NH₂, or OH;
R of Formula I is HO₂C(CH₂)ₙ₁CO-(AA11)ₙ₂-(PEGₙ₃(CH₂)ₙ₄CO)ₙ₅-;
wherein, R is not HO₂C(CH₂)ₙ₁CO-(AA11)ₙ₂-(PEG₂CH₂CO)₂-;
n1 is an integer selected from 10 to 20,
n2 is an integer selected from 1 to 5,
n3 is an integer selected from 1 to 30,
n4 is an integer selected from 1 to 5,
n5 is an integer selected from 1 to 5,
n6 is an integer selected from 1 to 10; and
AA11 is selected from the group consisting of yGlu, εLys, β-Ala, γ-aminobutyric acid, and 5-Ava.

2. The compound according to claim 1, wherein the compound is in a form selected from the group consisting of a pharmaceutically acceptable salt thereof, a solvate thereof, a chelate thereof, a non-covalent complex thereof, a prodrug thereof, and a mixture thereof.

3. Use of the compound according to claim 1 in the manufacture of a long-acting GLP-1/GLP-2 dual agonist.

4. Use of the compound according to claim 1 in the manufacture a medicament for treating a disease, wherein the disease is selected from the group consisting of type II diabetes, impaired glucose tolerance, type I diabetes, obesity, hypertension, metabolic syndrome, dyslipidemia, cognitive disorder, atherosclerosis, myocardial infarction, coronary heart disease, cardiovascular disease, stroke, inflammatory bowel syndrome, irritable bowel syndrome, indigestion or gastric ulcer, liver fibrotic disease, pulmonary fibrotic disease and a combination thereof.

5. A pharmaceutical composition comprising the compound according to claim 1.
